# EUROPEAN PATENT SPECIFICATION

(11) **EP 1 951 325 B1**
(45) Date of publication and mention of the grant of the patent: **22.12.2010**
(21) Application number: 06786038.7
(22) Date of filing: 29.06.2006
(51) Int. Cl.: A61L 9/16, A61L 9/18, A61L 101/22, A61L 101/36

(54) **METHOD OF SANITIZING A SHOPPING CART**
VERFAHREN ZUR DESINFEKTION EINES EINKAUFSWAGENS
PROCEDE DE DESINFECTION D'UN CADDIE

(30) Priority: 19.10.2005 US 254548
(43) Date of publication of application: 06.08.2008
(73) Proprietor: Purecart Systems, LLC, Green Bay WI 54301 (US)
(72) Inventor: SCHWEI, Mark, C., Greenville, WI 54942 (US); TAYLOR, Jeffrey, J., Green Bay, WI 54313 (US); WEYERS, Robert, J., Green Bay, WI 54313 (US); LENHART, John, G., Green Bay, WI 54302 (US)
(74) Representative: Hackett, Sean James
(86) International application number: PCT/US2006/025700
(87) International publication number: WO 2007/046894

(56) References cited:
- EP-A- 1 138 335
- WO-A-95/28471
- US-A1- 2005 214 159
- US-A1- 2005 217 701

## Description

The present invention generally relates to a system and method for sanitizing shopping carts and other similar transportation-related devices. More specifically, the present invention relates to a system that sanitizes shopping carts and devices designed to be in contact with humans by utilizing a sanitizing fluid that does not need to be rinsed from the devices and dries quickly.

Presently, supermarkets, grocery stores and similar shopping establishments provide shopping carts for the customer's convenience. The carts are adapted to hold the customer's selected purchases and thus often contain food, including liquids, which at times are spilled or come in contact with the cart surfaces. Additionally, many of the customers using the shopping carts have small children who are carried in the carts. These small children often place their mouths and hands on the shopping cart handles. Recently, numerous studies have been published that indicate that a large amount of bacteria and microorganisms, including viruses, on the handles and other areas of shopping carts.

Clearly, it is recognized as good sanitation practice to have shopping carts cleaned at regular periodic intervals to reduce the levels of microorganisms and bacteria that are living on the areas of the shopping cart that come into contact with a customer. Presently, federal reports have been generated that highlight the amount of bacteria and microorganisms existing on grocery carts. Failure to thoroughly clean such shopping carts could reduce the level of customers to a particular store in the future if customers are alerted to the presence of unsanitary, soiled carts at the store. Likewise, if a store periodically cleaned and sanitized the shopping carts, the store could advertise such a fact, which would greatly enhance the customer comfort level and eventually business at the store.

The standard practice at most stores that utilize shopping carts is to very infrequently clean the shopping carts manually by the use of a high pressure hot water wand including a detergent. Once the detergent is applied to the shopping cart, a hot water rinse is used to remove the detergent from the shopping carts. Unfortunately, the manual washing of carts is inefficient since it requires a great deal of paid employee time to wash the numerous carts present at a store. Further, the use of high pressure washing equipment and detergent creates a mess in the parking lot of the store, which makes the manual washing of the shopping carts even more undesirable. In fact, it may not be possible to clean these carts in colder climates during cold and flu season due to environmental and safety concerns.

Although the use of a high pressure hot water wand including detergent is effective at removing debris from the shopping carts, detergent is not effective at eliminating the bacteria and microorganisms included on the shopping cart. Further, the type of detergent used to clean the shopping carts must be approved for use around food items, since the shopping carts are used within the grocery store and may contact the outer surface of food items.

In addition to shopping carts, other transportation devices that come into contact with humans also need to be sanitized to eliminate bacteria and microorganisms, including viruses. Such transportation devices may include buses, amusement park rides, automobiles and other similar durable devices that are owned by a business and repeatedly used by multiple customers. Since customers contact various different surfaces of the transportation-related devices, it is desirable for the owner/operator of the business to be able to sanitize the transportation-related devices to prevent the transfer of bacteria and microorganisms, such as viruses, from one customer to another.

Therefore, a current need exists for a sanitizing system that effectively reduces the levels of bacteria and microorganisms on transportation- related devices, such as shopping carts, and utilizes an effective sanitizing fluid that is safe for human contact. A further need exists for a cart sanitizing system that is compact in size, easily movable and minimizes the capital expenditure required by the owner.
US 2005/214159 discloses a method of sanitizing shopping carts using a fluid that contains a sanitizing agent and a surfactant mixed in water. EP 1,138,335 discloses use of a mixture of peracetic acid with hydrogen peroxide to form a disinfecting solution for use in cleaning endoscopes.

### SUMMARY OF THE INVENTION

According to the present invention there is provided a method of sanitizing shopping carts as defined in claim 1.

The present invention relates to a method for applying a sanitizing fluid to a transportation-related device, such as a shopping cart, such that the shopping cart can be sanitized. An associated sanitizing system allows a shopping cart to be quickly and easily sanitized without requiring time consuming drying and rinsing procedures.

The sanitizing system includes a cart enclosure designed to receive the shopping cart being cleaned. The cart enclosure includes a floor, a pair of spaced side walls, a first end wall having an entrance and a second end wall having an exit.

The cart enclosure includes a spray arch positioned within the enclosure between the entrance and the exit. The spray arch includes multiple nozzles spaced around the arch, including a floor-mounted nozzle, to provide complete spray coverage when a shopping cart is beneath the spray arch. The spray arch is in fluid communication with a source of pressurized sanitizing fluid such that the sanitizing fluid can be sprayed onto the shopping cart through the spray nozzles. Preferably, each of the spray nozzles includes a diaphragm check valve such that the check valves prevent a discharge of sanitizing fluid when the pressure of the fluid supplied to the nozzles falls below a threshold pressure.

The pressurized sanitizing fluid is supplied to the spray arch from a pressure tank coupled to the spray arch through the control valve. The pressure tank includes a supply of sanitizing liquid at a desired pressure. The pressure within the pressure tank is created by a pump positioned between a supply tank and the pressure tank. When the pressure within the pressure tank falls below a minimum value, the pump introduces an additional supply of the sanitizing fluid into the pressure tank from the supply tank. In this manner, the pressure pump maintains the pressure within the pressure tank by supplying an additional sanitizing fluid.

The supply tank coupled to the pressure pump stores a supply of sanitizing fluid that is created through a chemical make-down system. The chemical make-down system mixes a desired supply of a surfactant and a desired supply of a sanitizing agent into a flow of fresh water to create the sanitizing fluid. The sanitizing agent within the sanitizing fluid allows the sanitizing fluid to sanitize the shopping cart while the surfactant reduces the surface tension of the water, which aids in the rapid drying of the shopping cart after the sanitizing fluid has been applied.

Preferably, the mixing device includes a surfactant inlet tube and a chemical inlet tube each having an inlet opening extending into a flow of fresh water. As the fresh water passes over the inlet openings for both the surfactant tube and the chemical tube, the flow of water draws both the surfactant and the sanitizing agent into the flow of water. The amount of surfactant and sanitizing agent drawn into the flow of water is dictated by the size of each of the respective inlet openings. The sanitizing liquid created by the chemical malce-down system is stored in the supply tank. The supply tank includes multiple level sensors that maintain the volume of sanitizing fluid in the supply tank between an upper level and a lower level where the stored supply of sanitizing fluid is created by the mixing device.

The sanitizing agent utilized to form the sanitizing fluid includes a mixture of peroxyacetic acid, hydrogen peroxide, acetic acid and carrier, such as water. In the most preferred embodiment of the invention, the concentrated sanitizing agent includes about 5 to 6 weight-% peroxyacetic acid, about 26 to 28 weight-% hydrogen peroxide and about 7 to 8 weight-% acetic acid. The concentrated sanitizing agent is injected into the flow of water such that the sanitizing agent exists at a ratio of approximately 40:1 to 1000:1. Most preferably, the sanitizing agent is injected at a ration of about 250:1.

The sanitizing fluid also includes the injected surfactant which aids in the drying process of the shopping cart after the application of the concentrated sanitizing fluid. Preferably, the surfactant is injected into the flow of water at a ratio of between 100:1 to 2000:1, and most preferably at a ratio of 1700:1. After both the sanitizing agent and the surfactant are injected into the flow of water, the created sanitizing fluid is stored in a pressurized container for application to the shopping cart using a spraying process. Preferably, the sanitizing fluid is applied to the shopping cart at ambient temperature and the shopping cart is allowed to air dry after application of the sanitizing fluid.

In addition to utilizing the sanitizing fluid to sanitize shopping carts, the sanitizing fluid can also be used to sanitize other types of transportation devices that come into contact with humans. The use of the sanitizing fluid on such transportation devices reduces the viral count on the transportation devices such that the transportation devices can be used by subsequent users with a reduced risk of viral transfer from the transportation device.

### BRIEF DESCRIPTION OF THE DRAWINGS

The drawings illustrate an apparatus for carrying out the invention. In the drawings;

Figure 1 is a perspective view of the shopping cart sanitizing system suitable for carrying ont the invention;

Figure 2 is an end view of the sanitizing system shown in Figure 1;

Figure 3 is a fluid flow diagram illustrating the creation of the sanitizing fluid and the application of the sanitizing fluid through the spray arch and manual spray wand; and

Figure 4 is a schematic illustration of the chemical make-down system for use in the method according to the invention.

### DETAILED DESCRIPTION OF THE PREFERRED EMBODIMENT

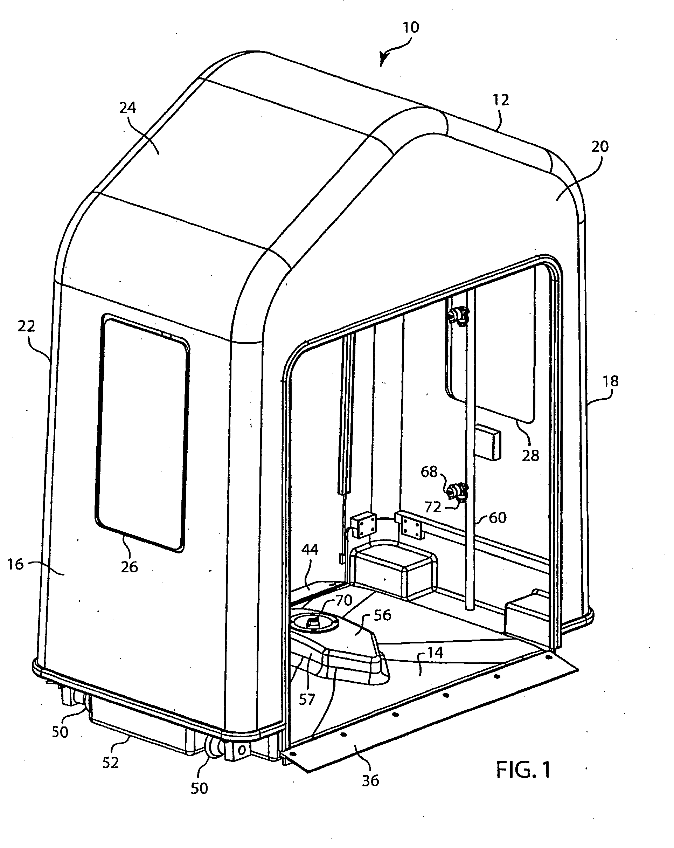

Referring first to Figure 1, thereshown is a cart sanitizing system 10. The cart sanitizing system 10 is configured to receive one or more shopping carts and apply a sanitizing fluid to the shopping carts to substantially reduce the number of microorganisms and bacteria present on the shopping cart. Throughout the following description, the cart sanitizing system is described as sanitizing a shopping cart. The term sanitizing refers to substantially reducing the number of bacteria and other microorganisms, such as viruses, and does not require the complete elimination of such organisms. The cart sanitizing system 10 generally includes a cart enclosure 12 having a floor 14, a pair of spaced side walls 16,18, a first end wall 20 and a second end wall 22. The top of the cart enclosure 12 includes a generally curved top wall 24.

In Figure 1, the pair of side walls 16, 18, the first and second end walls 20,22 and the generally curved top wall 24 are formed by a molded, unitary enclosure, preferably formed from fiberglass. Each of the side walls 16, 18 includes a viewing window 26,28 to allow the progress of the washing procedure to be viewed from the exterior of the molded cart enclosure 12. Although a unitary molded enclosure is shown in Fig. 1 as being the preferred embodiment of the invention, the enclosure could be formed utilizing other materials, such as polycarbonate sheets and aluminum support beams.

The shopping cart enclosure 12 includes an entrance ramp 36 that contacts the ground or floor and provides a ramp for a shopping cart to be pushed upward through the entrance and onto the floor 14 of the enclosure 12. The shopping cart enclosure 12 further includes an exit ramp 44 mounted to the enclosure 12 near the second end wall 22 that is in contact with the ground or floor.

The cart enclosure 12 includes two pairs of caster wheels 50 mounted near both the entrance and exit of the cart enclosure 12. The caster wheels 50 include a hinge mechanism that allow the caster wheels to be raised and lowered to permit movement of the cart enclosure. When the caster wheels 50 are in their retracted position as shown in Fig. 1, the bottom surface 52 of the cart enclosure 12 contacts the ground to prevent movement of the cart enclosure. When the cart enclosure 12 needs to be moved, each of the pair caster wheels 50 is rotated to its extended position, which allows the shopping cart enclosure to be rolled to a desired location.

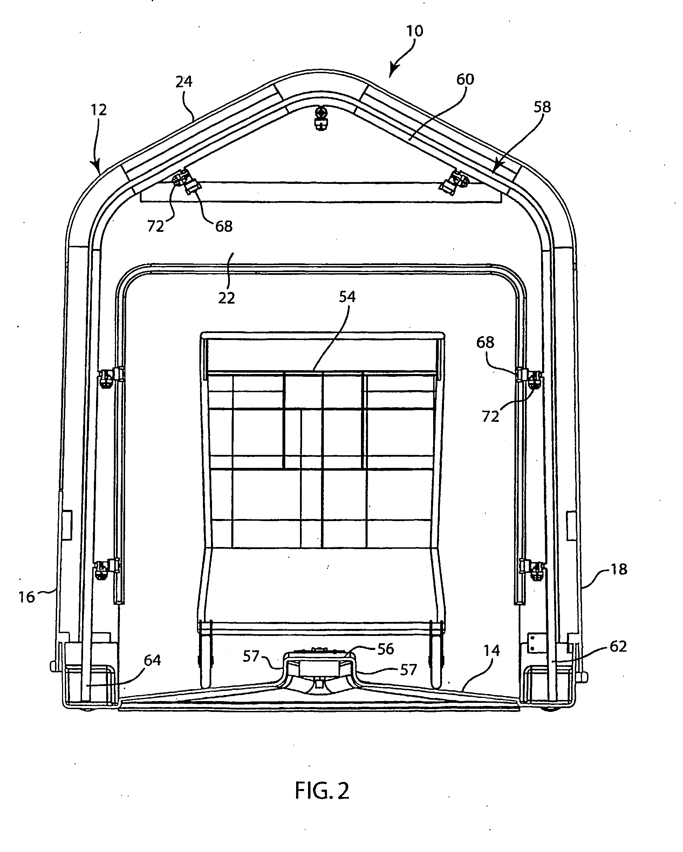

Referring now to Figure 2, the cart enclosure 12 includes a centering block 56 mounted to the floor 14 of the cart enclosure. The centering block 56 includes angled side surfaces that contact the front wheels of the shopping carts to aid in guiding the shopping cart 54 through the cart enclosure 12. The distance between the outer edges 57 of the centering block 56 is adjustable depending upon the type of shopping cart 54 being sanitized.

The cart enclosure 12 includes a spray arch 58 that is spaced slightly inward of the outer walls of the cart enclosure. The spray arch 58 includes a fluid conduit 60 that extends from an inlet end 62 to the termination end 64. The inlet end 62 connects to a supply source for the sanitizing fluid. In the embodiment of the invention illustrated, the fluid conduit 60 is formed from PVC, although other materials are contemplated as being within the scope of the invention.

The spray arch 58 includes a series of nozzles 68 spaced along the length of the spray arch. As best seen in Figure 2, the spray arch 58 includes seven individual nozzles 68 spaced around the shopping cart 54 when the shopping cart is positioned within the shopping cart enclosure 12. In addition to the seven nozzles 68 spaced around the shopping cart, the sanitizing system includes a floor mounted nozzle 70, as best seen in Fig. 1. The combination of the seven nozzles 68 and the floor mounted nozzle 70 provide a complete spray pattern that covers the entire shopping cart 54 when the shopping cart 54 passes beneath the spray arch 58.

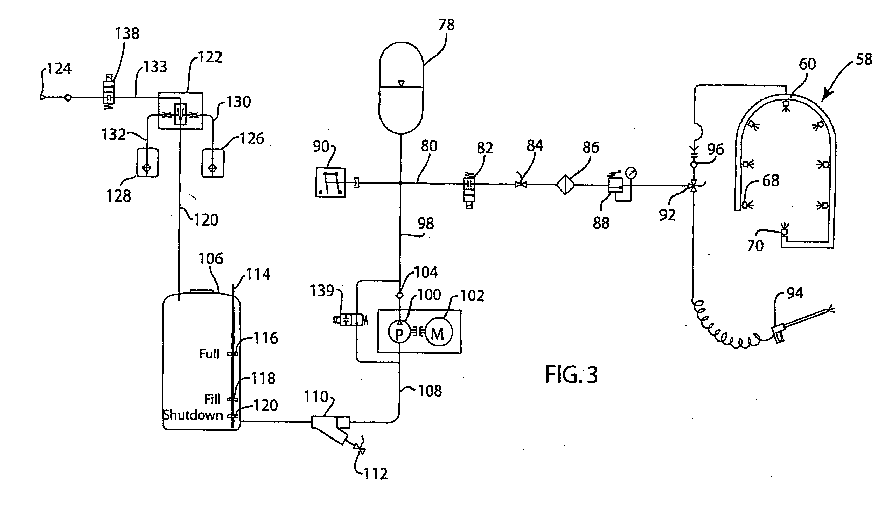

Each of the spray nozzles 68 is coupled to the fluid conduit 60 by a nozzle body 72 that includes a diaphragm check valve. In operation, the check valve of the nozzle body 72 allows fluid to reach the spray nozzle 68 only when the pressure of the fluid being distributed exceeds a threshold pressure. The check valves open when the pressure of the fluid within the fluid conduit 60 exceeds between 5 and 7 psi. Thus, when the pressure of the liquid within the fluid conduit 60 falls below the threshold value, the diaphragm check valve prevents liquid from being discharged through the associated nozzle 68. In the embodiment of the invention illustrated in Figure 3, each nozzle 68 includes a spray tip that delivers flat, angular spray patterns, although other spray patterns are contemplated.

Although not shown, the shopping cart enclosure 12 includes a drain mounted to the floor 14. The drain includes a drain opening that receives the excess fluid applied to the shopping cart and directs the excess fluid to a drain tube that can be connected to a drain hose such that the used sanitizing fluid can be fed to a discharge location, such as a sewer or drain.

Referring now to Figure 3, the fluid flow system and circuit for the cart sanitizing system will be described. As illustrated, the fluid conduit 60 of the spray arch 58 is shown including the spray nozzles 68 and the floor mounted nozzle 70. The spray arch 58 is supplied with a pressurized source of sanitizing fluid from a pressure tank 78 through the fluid conduit 80. The fluid conduit 80 includes a control valve 82 that is operable between an open and a closed position. The control valve 82 controls the supply of pressurized sanitizing fluid to the spray arch 58. In accordance with the present invention, the control valve 82 can be controlled by an operator using a remote control device.

In addition to the control valve 82, the fluid conduit 80 includes the manual shutoff valve 84, an inline strainer 86 and a pressure reducing valve 88. The pressure reducing valve 88 reduces the pressure from the pressure tank 78 down to a constant, selected value. The pressure reducing valve 88 reduces the pressure of the sanitizing fluid to 40 psi. The charging of the pressure tank 78 is controlled by a pressure switch 90 to regulate the pressure within the tank to a range between 40 and 60 psi.

The fluid conduit 80 further includes a selection valve 92 that is operable to divert the flow of pressurized sanitation fluid between either the spray arch 58 or a manual spray wand 94. When the pressurized fluid is diverted to the manual spray wand 94, a user can spray the sanitizing fluid onto carts or other transportation-related devices when the devices are not contained within the cart enclosure. A check valve 96 prevents the reverse flow of fluid from the spray arch 58.

The pressure tank 78 has a fifteen gallon capacity and is fed with sanitizing fluid through a filling conduit 98 by a pump 100 driven by motor 102. The operation of the pump 100 and motor 102 is controlled by the pressure switch 90 such that the pressure within the pressure tank is maintained between 40 and 60 psi. In the embodiment of the invention illustrated, the pump 100 is a magnetic driven, positive displacement pump with a capacity of approximately 2.2 gpm at 40 to 60 psi. A check valve 104 prevents the reverse flow of fluid within the filling conduit 68.

As illustrated in Figure 3, the sanitizing fluid used to fill the pressure tank 78 is drawn from a bulk storage tank 106 through the make-up conduit 108. The make-up conduit 108 includes a strainer 110 having a manual drain 112. As the pressure tank 78 discharges fluid to the spray arch 58, the make-up fluid is supplied to the pressure tank 78 by the pump 100 through the make-up conduit 108 from the storage tank 106. The storage tank 106 is a 75 gallon tank having a sensing probe 114. The sensing probe 114 includes three sensors for detecting the level of fluid within the storage tank 106. The first sensor 116 defines a maximum fill level for the storage tank 106. The second sensor 118 defines a lower level for the liquid within the tank, while the third sensor 120 is a shutdown sensor that prevents operation of the system should the liquid level within the storage tank 106 fall below this absolute minimum.

The storage tank 106 is fed with the sanitizing fluid by the fluid conduit 120. The fluid conduit 120 receives the sanitizing fluid from a mixing device 122. The mixing device 122 receives a supply of fresh water from fresh water inlet 124 and injects a surfactant from a surfactant tank 126 and a sanitizing agent from a chemical tank 128. The surfactant tank 126 is a one gallon capacity tank while the chemical tank 128 has a five gallon capacity. The surfactant tank 126 is in fluid communication with the mixing device 122 by a surfactant tube 130, while the chemical tank 128 is in fluid communication with the mixing device 122 through a chemical tube 132.

### Sanitizing Anent

The sanitizing agent contained within the chemical tank 128 is a concentrated mixture that includes peroxyacetic acid, hydrogen peroxide, acetic acid and a carrier such as water. The concentrated sanitizing agent including this combination of components has been found to be particularly desirable in reducing the amount of bacteria and microorganisms, including viruses, on the handles and other areas of shopping carts. An example of sanitizing fluid including these components is sold under product name PERASAN® A, available from Enviro-Tech Chemical Service, Inc. of Modesto, California.

In the preferred embodiment of the invention, the concentrated sanitizing agent includes peroxyacetic acid in concentration levels of about 1 to about 20 weight-%. Hydrogen peroxide is present in the sanitizing agent at concentration levels of about 2 to about 40 weight-%, while the acetic acid is present in the sanitizing agent at levels of about 5 to about 50 weight-%.

In the most preferred embodiment of the invention, the concentrated sanitizing agent includes peroxyacetic acid in the concentrations of about 5 to about 6 weight-%. Hydrogen peroxide is present in the sanitizing agent at concentrations levels of about 26 to about 28 weight-%, while the acetic acid is present in the sanitizing agent at levels of about 7 to about 8 weight-%. The presence of hydrogen peroxide in combination with the peroxyacetic acid provides a high level of antimicrobial action against microorganisms, even in the presence of high loadings. Further, the use of hydrogen peroxide is particularly advantageous since this compound is acceptable for use in food contact surfaces. For example, the combination of peroxyacetic acid and hydrogen peroxide results in acetic acid, water and oxygen, upon decomposition. All of these constituents are food product compatible.

In addition to the sanitizing agent, a rinse agent, such as a surfactant is also mixed into the flow of water prior to the sanitizing fluid being stored in the storage tank 106. The surfactant is added to the sanitizing fluid to aid in rinsing and to promote sheeting. Generally, any number of surfactants may be used while operating within the scope of the present invention. For example, the surfactant may be a non ionic, anionic, cationic or amphoteric surfactant. As an example, the surfactant Pro RINSE aid available from Pro Chemicals of Green Bay, Wisconsin may be utilized in forming the sanitizing fluid of the present invention.

In a preferred embodiment of the invention, a fragrance is also combined with the surfactant in the surfactant tank 126. The fragrance is utilized to provide a more pleasurable smell to the user. Examples of the fragrance include lemon, orange or other citrus fragrances. The citrus fragrance provides the user of the shopping cart with the assurance that the shopping cart has been cleaned. However, it is contemplated that the sanitizing fluid could be created without the use of any type of fragrance.

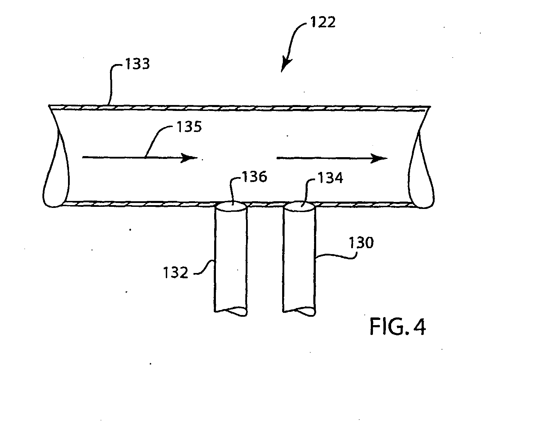

Referring now to Figure 4, thereshown is a schematic illustration of the mixing device 122. The mixing device 122 includes a fresh water conduit 133 that receives the flow of fresh water from the inlet. As the flow of fresh water 135 flows through the fresh water conduit 133, the flow of water passes over an inlet opening 134 of the surfactant tube 130 and an inlet opening 136 of the chemical tube 132. As the flow of fresh water 135 passes over the inlet openings 134 and 136, the flow of fresh water draws both the surfactant and the sanitizing agent into the fresh water conduit 133. In this manner, the mixing device 122 creates the desired mixture for the sanitizing fluid as it exits the mixing device 122 and is supplied to the storage tank 106.

The concentration of both the surfactant and the sanitizing agent within the sanitizing fluid is controlled by the diameter of the inlet openings 134 and 136. Thus, to modify the amount of either the surfactant or the sanitizing agent within the sanitizing fluid, the diameter of the inlet openings 134,136 can be modified. Preferably, the inlet openings 134,136 are contained in replaceable sections of the surfactant tube 130 and the chemical tube 132. Thus, the replaceable sections of the tubes can be modified if the concentration of the sanitizing fluid needs to be changed based upon the types of chemicals being used.

In a preferred embodiment of the invention, the concentrated sanitizing agent is supplied into the flow of water such that the ratio of sanitizing agent to water within the sanitizing fluid is between 40:1 and 1000:1. The most preferred ratio of the sanitizing agent to water has been found to be 250:1 (0.4%). However, it should be understood that the lower the ratio of the sanitizing agent to water, the more effective the sanitizing fluid will be. The lower ratio will increase the cost to sanitize each cart, since more of the sanitizing agent will be utilized for each wash procedure. The ratios set forth above were found to be the most cost effective while providing the required sanitizing performance. As discussed above, the diameter of the inlet opening 136 of the chemical tube 132 controls the flow of the concentrated sanitizing agent into the flow of water. In addition to the sanitizing agent, surfactant is supplied into the flow of water to create the sanitizing fluid at a ratio of 100:1 to approximately 2000:1. The most preferred ratio of the surfactant to water has been found to be 1700:1 (0.06%). Once again, the ratio of the surfactant to water could be lower, resulting in more effective rinsing at the expense of increased cost. The ratios set for were found to be the most cost effective while providing the required performance.

As described above, the sanitizing fluid applied to the shopping carts include both the surfactant and the concentrated sanitizing agent where the sanitizing agent includes peroxyacetic acid, hydrogen peroxide and acetic acid. Through testing, it has been found that the sanitizing fluid is effective at reducing the bacterial and microorganism levels, including viral loads, on a non-porous surface a significant amount, as listed in the test results below.

### Viral Test Results

### INFLUENZA VIRUS ASSAYS

**Viral Strain: A/Puerto Rico/8/34** - human origin H1N1 virus (represents circulating strains)
**Cells: Madin Darby canine kidney cells**

### Procedure:

1. MDCK cells were seeded at 2 x 10⁴ cells per well in a 96-well flat bottom tissue culture plate and allowed to incubate overnight.
2. Growth media was removed and cell monolayers were washed 2X with sterile PBS pH 7.4. Test samples were diluted from 1:10 to 1:1,000,000 in MEM containing 1% BSA and 1 µg/ml TPCK trypsin (required for viral growth), and 100 µl was added to cells in triplicate.
3. Cells were incubated for 3 days at 37C, 5%CO₂ and then visually scored for cell death or death confirmed by MTS assay (Promega).

### *Level of Detection is 10^{0.5}

Representative results: Demonstrates that virus was completely inactivated with 8 minute incubation. Partial inactivation was observed within 5 minutes.

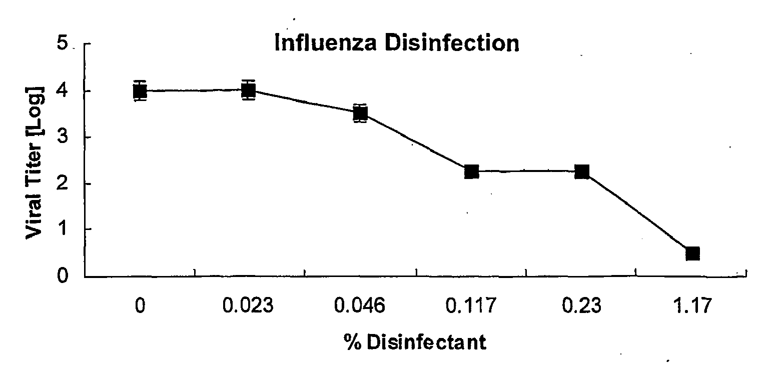

### ADENOVIRUS ASSAY

Adenovirus is one of the most common human cold viruses. This particular strain is wide-spread in children, highly contagious, and notoriously difficult to inactivate. **Viral Strain: Adenovirus Strain 2** - human origin obtained from the ATCC (VR-846) specifically for these studies.

### Cells: A549 human lung cells

Procedure:
1. A549 cells were seeded at 5 x 10⁴ cells per well in a 96-well flat bottom tissue culture plate and allowed to incubate overnight.
2. Growth media was removed and cell monolayers were washed 2X with sterile PBS pH 7.4. Test samples were diluted from 1:10 to 10⁸ (log dilutions) in MEM containing 2% fetal bovine sera and 100 µl was added to cells in triplicate.
3. Cells were incubated for 4 to 6 days at 37C, 5%CO₂ and then visually scored for cell death.

### *Level of Detection is 10^{0.5}

Representative results: Demonstrates that virus is completely inactivated with five minute incubation.

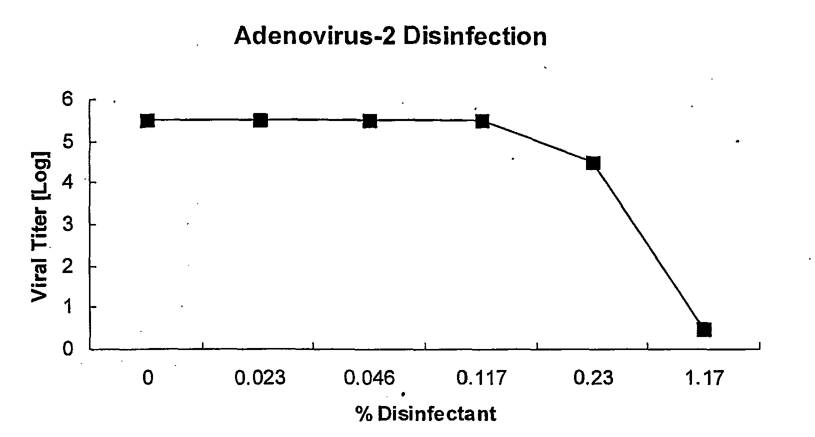

As the above test results indicate, the sanitizing fluid is effective at reducing the viral counts on surfaces to which the sanitizing fluid is applied. Thus, the sanitizing fluid can be applied to surfaces that come into contact with humans, such as those on durable transportation devices utilized by a business owner. As an example, the sanitizing fluid can be utilized to reduce the viral counts on transportation-related devices, such as amusement park rides, shopping carts, mass transportation device and other similar devices that come into contact with humans and are reused by different customers. The sanitizing fluid described above effectively reduces the viral counts on surfaces that come into contact with humans by utilizing the sanitizing fluid, which is safe for human contact. Further, the sanitizing fluid can be safe for human contact immediately upon application and thus presents no health risks immediately following usage.

As can be understood in Figs. 1 and 2, the sanitizing fluid is preferably sprayed onto the shopping cart at generally ambient temperatures utilizing a pressurized spray conduit and the series of spray nozzles. The position of the spray nozzles 68 are optimized to provide complete coverage over the entire shopping cart. However, since the handle of the shopping cart typically has the largest concentration of microorganisms, the spray nozzles are particularly concentrated to provide complete coverage over the handle of the shopping cart.

Once the shopping cart has left the cart enclosure, the shopping cart is allowed to air dry. The surfactant contained within the sanitizing fluid aids in the drying process of the shopping cart after the application of the sanitizing fluid. The surfactant prevents drips by spreading the sanitizing fluid into a thin film that also enhances the coverage of the sanitizing fluid, which also increases the effectiveness of the fluid.

The composition of the sanitizing fluid may also contain a chelating agent that aids in the reducing of harmful effects of hardness components in the service water. The typical harmful effects of calcium, magnesium, iron, manganese etc. present in service water can interfere with the action of either the washing compositions or rinsing compositions and can tend to decompose the active sanitizer materials. The chelating agent can thus effectively increase the sanitizing performance. Various types of chelating agents can be utilized with the sanitizing fluid of the present invention.

Referring back to Figure 3, a water fill valve 13 8 is positioned to control the flow of fresh water through the fresh water conduit 133. The water fill valve 138 is operatively connected to the first sensor 116 and the second sensor 118. Thus, when the level of fluid within the storage tank 106 rises above the first sensor 116, the water fill valve 138 is closed to present any further supply of the sanitizing fluid. Likewise, when the liquid level in the tank 106 falls below the second sensor 118, the water fill valve 13 8 is opened to create an additional supply of the sanitizing fluid. As can be easily understood by the above description, the mixing device 122 creates the sanitizing fluid as the sanitizing fluid is required to fill the storage tank 106. Thus, the sanitizing fluid is created on the fly and only when desired.

The sanitizing fluid includes both the surfactant from the surfactant tank 126 and the sanitizing agent from the chemical tank 128. The surfactant functions to reduce the surface tension of the fresh water supply such that when the sanitizing fluid is applied to the shopping cart, the sanitizing fluid resists beading and dries more quickly. The sanitizing agent is selected in accordance with the present invention to provide a high level of sanitation to the shopping cart. In a preferred embodiment of the invention, the sanitizing liquid is a chemical agent that has been found to be food safe and does not require rinsing from the shopping carts. However, other types of sanitizing agents could be utilized while operating within the scope of the present invention.

When the cart sanitizing system shown in Fig. 3 is turned off, the dump valve 139 is opened such that the pressurized supply of sanitizing fluid from the pressure tank 78 is discharged into the storage tank 106. As illustrated in Fig. 3, the position of the first sensor 116 is selected such that when the storage tank 106 is filled to its maximum height, the storage tank 106 can still accept the full volume of the pressure tank 78.

## Claims

1. A method of sanitizing shopping carts (54), comprising:
storing a concentrated sanitizing agent including peroxyacetic acid, hydrogen peroxide, acetic acid and a carrier in a first container (128);
storing the surfactant in a second container (126);
injecting the concentrated sanitizing agent and the surfactant into a flow of water to create a sanitizing fluid comprising peroxyacetic acid, hydrogen peroxide, acetic acid, a surfactant and water for application directly to a shopping cart (54);
applying the sanitizing fluid to the shopping cart to reduce the microbial population on the shopping cart; and
allowing the shopping cart (54) to air dry after application of the sanitizing fluid.

2. The method of claim 1 wherein the sanitizing fluid is applied by an automated spraying machine (10).

3. The method of claim 1 wherein the sanitizing fluid further comprises a fragrance.

4. The method of claim 1 wherein the sanitizing fluid comprises:
about 1 to about 20 weight-% peroxyacetic acid;
about 3 to about 40 weight-% hydrogen peroxide; and
about 5 to about 50 weight-% acetic acid.

5. The method of claim 1 wherein the sanitizing fluid comprises:
about 5 to about 6 weight-% peroxyacetic acid;
about 26 to about 28 weight-% hydrogen peroxide; and
about 7 to about 8 weight-% acetic acid.

6. The method of claim 1 wherein the sanitizing fluid is applied to the shopping cart (54) at ambient temperature.

7. The method of claim 1 wherein the sanitizing fluid is applied to only a handle of shopping cart (54).

8. The method of claim 1 wherein the sanitizing fluid further comprises a chelating agent.

9. The method of claim 1 wherein the concentrated sanitizing agent is injected into the flow of water at a ratio between 40:1 and 1000:1.

10. The method of claim 9 wherein the concentrated sanitizing agent is about 250:1.

11. The method of claim 9 wherein the surfactant is injected into the flow of water at a ratio of between 100:1 and 2000:1.

12. The method of claim 11 wherein the surfactant is injected into the flow of water at a range of about 1700:1.

13. The method of claim 5 wherein the concentrated sanitizing agent is injected into the flow of water at a ratio between 40:1 1 and 1000:1.

14. The method of claim 13 wherein the surfactant is injected into the flow of water at a ratio of between 100:1 1 and 2000:1.

## Patentansprüche

1. Verfahren zur Desinfektion von Einkaufswagen (54), das die folgenden Schritte aufweist:
Lagern eines konzentrierten Desinfektionsmittels, das Peroxyessigsäure, Wasserstoffperoxid, Essigsäure und eine Trägersubstanz in einem ersten Behälter (128) umfasst;
Lagern der oberflächenaktiven Substanz in einem zweiten Behälter (126);
Einspritzen des konzentrierten Desinfektionsmittels und der oberflächenaktiven Substanz in einen Wasserstrom, um ein Desinfektionsfluid herzustellen, das Peroxyessigsäure, Wasserstoffperoxid, Essigsäure, eine oberflächenaktive Substanz und Wasser für eine direkte Aufbringung auf einen Einkaufswagen (54) aufweist;
Aufbringen des Desinfektionsfluids auf den Einkaufswagen, um die mikrobielle Population auf dem Einkaufswagen zu verringern; und
Zulassen, dass der Einkaufswagen (54) nach der Aufbringung des Desinfektionsfluids an der Luft trocknet.

2. Verfahren nach Anspruch 1, bei dem das Desinfektionsfluid mittels einer automatischen Spritzmaschine (10) aufgebracht wird

3. Verfahren nach Anspruch 1, bei dem das Desinfektionsfluid außerdem einen Duft aufweist.

4. Verfahren nach Anspruch 1, bei dem das Desinfektionsfluid aufweist:
etwa 1 bis etwa 20 Gew.-% Peroxyessigsäure;
etwa 3 bis etwa 40 Gew.-% Wasserstoffperoxid; und
etwa 5 bis etwa 50 Gew.-% Essigsäure.

5. Verfahren nach Anspruch 1, bei dem das Desinfektionsfluid aufweist:
etwa 5 bis etwa 6 Gew.-% Peroxyessigsäure;
etwa 26 bis etwa 28 Gew.-% Wasserstoffperoxid; und
etwa 7 bis etwa 8 Gew.-% Essigsäure.

6. Verfahren nach Anspruch 1, bei dem das Desinfektionsfluid auf den Einkaufswagen (54) bei Umgebungstemperatur aufgebracht wird.

7. Verfahren nach Anspruch 1, bei dem das Desinfektionsfluid nur auf einen Griff des Einkaufswagens (54) aufgebracht wird.

8. Verfahren nach Anspruch 1, bei dem das Desinfektionsfluid außerdem ein eine Chelatbildung bewirkendes Mittel aufweist.

9. Verfahren nach Anspruch 1, bei dem das konzentrierte Desinfektionsmittel in den Wasserstrom mit einem Verhältnis zwischen 40:1 und 1000:1 eingespritzt wird.

10. Verfahren nach Anspruch 9, bei dem das konzentrierte Desinfektionsmittel etwa 250:1 beträgt.

11. Verfahren nach Anspruch 9, bei dem die oberflächenaktive Substanz in den Wasserstrom mit einem Verhältnis von zwischen 100:1 1 und 2000:1 eingespritzt wird.

12. Verfahren nach Anspruch 11, bei dem die oberflächenaktive Substanz in den Wasserstrom in einem Bereich von etwa 1700:1 eingespritzt wird.

13. Verfahren nach Anspruch 5, bei dem das konzentrierte Desinfektionsmittel in den Wasserstrom mit einem Verhältnis zwischen 40:1 und 1000:1 1 eingespritzt wird.

14. Verfahren nach Anspruch 13, bei dem die oberflächenaktive Substanz in den Wasserstrom mit einem Verhältnis von zwischen 100:1 1 und 2000:1 1 eingespritzt wird.

## Revendications

1. Procédé de désinfection de caddies (54), comprenant les étapes ci-dessous:
stockage d'un agent désinfectant englobant de l'acide peroxyacétique, du peroxyde d'hydrogène, de l'acide acétique et un véhicule dans un premier récipient (128) ;
stockage d'un agent de surface dans un deuxième récipient (126);
injection de l'agent désinfectant concentré et de l'agent de surface dans un écoulement d'eau pour produire un fluide désinfectant comprenant de l'acide peroxyacétique, du peroxyde d'hydrogène, de l'acide acétique, un agent de surface et de l'eau, en vue d'une application directe sur un caddie (54) ;
application du fluide désinfectant sur le caddie pour réduire la flore microbienne sur le caddie ; et
séchage à l'air du caddie (54) après l'application du fluide désinfectant.

2. Procédé selon la revendication 1, dans lequel le fluide désinfectant est appliqué par une machine de pulvérisation automatique (10).

3. Procédé selon la revendication 1, dans lequel le fluide désinfectant comprend un parfum.

4. Procédé selon la revendication 1, dans lequel le fluide désinfectant comprend :
environ 1 à environ 20% en poids d'acide peroxyacétique ;
environ 3 à environ 40% en poids de peroxyde d'hydrogène;
environ 5 à environ 50 % en poids d'acide acétique.

5. Procédé selon la revendication 1, dans lequel le fluide désinfectant comprend :
environ 5 à environ 6% en poids d'acide peroxyacétique ;
environ 26 à environ 28% en poids de peroxyde d'hydrogène ; et
environ 7 à environ 8% en poids d'acide acétique.

6. Procédé selon la revendication 1, dans lequel le fluide désinfectant est appliqué sur le caddie (54) en présence de la température ambiante.

7. Procédé selon la revendication 1, dans lequel le fluide désinfectant est appliqué uniquement sur une poignée du caddie (54).

8. Procédé selon la revendication 1, dans lequel le fluide désinfectant comprend en outre un agent chélateur.

9. Procédé selon la revendication 1, dans lequel l'agent désinfectant concentré est injecté dans l'écoulement d'eau dans un rapport compris entre 40 :1 et 1000 :1.

10. Procédé selon la revendication 9, dans lequel l'agent désinfectant concentré est injecté dans un rapport d'environ 250 :1.

11. Procédé selon la revendication 9, dans lequel l'agent de surface est injecté dans l'écoulement d'eau dans un rapport compris entre 100 :1 et 2000 :1.

12. Procédé selon la revendication 11, dans lequel l'agent de surface est injecté dans l'écoulement d'eau dans un rapport d'environ 1700 :1.

13. Procédé selon la revendication 5, dans lequel l'agent désinfectant concentré est injecté dans l'écoulement d'eau dans un rapport compris entre 40 :1 et 1000 :1.

14. Procédé selon la revendication 13, dans lequel l'agent de surface est injecté dans l'écoulement d'eau dans un rapport compris entre 100 :1 et 2000 :1.
